Europäisches Patentamt

European Patent Office

Office européen des brevets

(19)

(11) Veröffentlichungsnummer: **0 051 747**
**A1**

(12) **EUROPÄISCHE PATENTANMELDUNG**

(21) Anmeldenummer: **81108004.3**

(22) Anmeldetag: **07.10.81**

(51) Int. Cl.³: **A 61 B 17/36**

(30) Priorität: **08.11.80 DE 3042202**

(43) Veröffentlichungstag der Anmeldung: **19.05.82**
**Patentblatt 82/20**

(84) Benannte Vertragsstaaten: **AT BE CH FR GB LI NL SE**

(71) Anmelder: **MESSER GRIESHEIM GMBH,**
**Patentabteilung Hanauer Landstrasse 330,**
**D-6000 Frankfurt/Main (DE)**

(72) Erfinder: **Volker, Wolfgang, Joachimstrasse 50,**
**D-4000 Düsseldorf (DE)**
Erfinder: **Weinknecht, Bernd, Hubertus-Strasse 10,**
**D-4044 Kaarst 2 (DE)**
Erfinder: **Jankowski, Detlef, Kortumstrasse 127,**
**D-4100 Duisburg 1 (DE)**

(54) **Gerät zur Entfernung von Warzen mit Flüssigstickstoff.**

(57) Bei einem Gerät zur Entfernung von Warzen mit Flüssigstickstoff ist ein metallischer Kühlstab (12) vorgesehen, in dessen Innerem ein Temperaturfühler (4) angeordnet ist. Durch eine Kabelverbindung wird der Temperaturfühler mit einem elektrischen Temperaturmeßgerät verbunden. Zur Warzenentfernung wird die Kabelverbindung gelöst und über den Kühlstab ein Isolierrohr geschoben, mit dem der Kühlstab ergriffen und die Behandlung durchgeführt wird. Eine Temperaturkontrolle während der Behandlung ist nicht möglich. Der behandelnde Arzt benötigt daher viel Erfahrung, um die Kältezufuhr möglichst optimal zu dosieren.

Um die Behandlung zu vereinfachen, wird der Kühlstab von einem pistolenähnlichen Griff (1) aufgenommen, in dem das Temperaturmeßgerät (5) angeordnet ist und auf dem sich die Temperaturanzeige (8, 9) befindet. Die Temperaturanzeige kann optisch oder akustisch erfolgen. Optimiert wird das Gerät durch eine auf dem Griff angeordnete Einrichtung (10) zur Zeitmessung.

EP 0 051 747 A1

– 1 –

MESSER GRIESHEIM GMBH           MG 1252

Kennwort: Cryo-Colt                    EM  980

Erfinder:  D.Jankowski                 Ordner:  A
           W.Volker
           B.Weinkecht

## Gerät zur Entfernung von Warzen mit Flüssigstickstoff

Die Erfindung betrifft ein Gerät zur Entfernung von
Warzen mit Flüssigstickstoff, welches im wesentlichen
aus einem metallischem Kühlstab besteht, in dessen Innerem ein Temperaturfühler angeordnet ist, welcher mit
einem elektrischen Temperaturmeßgerät verbindbar ist.

Die Entfernung von Warzen durch Einwirkung von flüssigem
Stickstoff ist seit Jahrzehnten bekannt. Bis heute hat
sich eine einfache Behandlungspraxis erhalten, bei der der
Patient mit einem in Flüssigstickstoff getauchten Wattestäbchen behandelt wird,  indem dieses mit Flüssigstickstoff getränkt und auf die Oberfläche der Warze aufgedrückt wird. Ein Nachteil dieser Behandlungweise ist die
ungenaue Dosierung und die geringe Kältekapazität des
Wattestäbchens.

Da die Behandlungsdauer meist ein bis zwei Minuten beträgt, muß das Wattestäbchen mehrfach erneut mit Flüssigstickstoff getränkt werden. Die hierbei unvermeidliche Unterbrechung der Kältezufuhr in die Warze hat sich als nachteilig erwiesen.

In neuerer Zeit ist man daher teilweise dazu übergegangen, die Entfernung von Warzen mit metallischen Kühlstäben vorzunehmen. Das Prinzip besteht darin, den metallischen Kühlstab - gewöhnlich aus Kupfer - in Flüssigstickstoff solange einzutauchen, bis er die Temperatur des flüssigen Stickstoffs angenommen hat. Danach wird der Kühlstab auf die Warze gedrückt. Zur Temperaturkontrolle befindet sich in der Spitze des Kühlstabes ein Temperaturfühler, der über eine Kabelverbindung an ein elektrisches Temperaturmeßgerät angeschlossen ist. Zur Behandlung wird die Kabelverbindung gelöst, der Kühlstab in ein Isolierrohr gesetzt und vom Arzt auf die Warze gedrückt. Sofern der metallische Kühlstab eine genügende Kältekapazität hat, ist eine Unterbrechung der Behandlung nicht erforderlich. Eine Temperaturkontrolle ist aber nicht möglich, es kann demnach passieren, daß sich der Kühlstab zu sehr erwärmt und keine ausreichende Kältezufuhr zur Warze mehr erfolgt. Es bedarf also erheblicher Erfahrung des behandelnden Arztes, um mit dem metallischen Kühlstab die gewünschte Kältemenge optimal zu dosieren. Eine Temperaturkontrolle wird hierbei gelegentlich durchgeführt, indem Thermoelmentnadeln unter die Behandlungsstelle eingestochen werden, eine Unannehmlichkeit, welche man dem Patienten möglichst ersparen will. Ferner ist es möglich, die Kabelverbindung zum Temperaturmeßgerät während der Behandlung beizubehalten. Hierdurch würde der Arzt jedoch ständig von der eigentlichen Behandlungsstelle abgelenkt. Außerdem ist es sehr umständlich, den Kühlstab mit aufrechterhaltener Kabelverbindung in ein Isolierrohr oder eine andere Haltevorrichtung einzulegen.

Der Erfindung liegt die Aufgabe zugrunde, ein im wesentlichen aus einem metallischem Kühlstab bestehendes Gerät zur Entfernung von Warzen mit Flüssigstickstoff zu schaffen, welches jegliche Kabelverbindung vermeidet und es dem behandelnden Arzt trotz ständiger Temperaturkontrolle gestattet, seine Aufmerksamkeit ungeteilt der eigentlichen Behandlung zu widmen.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Die Einrichtungen zur Temperaturkontrolle werden, sofern sie sich optischer Mittel bedienen, so auf dem Griff gemäß der Erfindung angeordnet, daß sie der behandelnde Arzt gleichzeitig mit dem zu behandelnden Objekt im Auge behalten kann. Erfolgt die Temperaturkontrolle durch akustische Hilfsmittel, so können diese auch an anderen Stellen des Griffes angebracht werden. Die Möglichkeiten zur Anzeige des Temperaturbereiches sind vielfältig. So kann z.B. eine zu hohe Temperatur durch eine rotblinkende Leuchtdiode, eine ausreichend tiefe Temperatur durch eine grünblinkende Leuchtdiode angezeigt werden. Auf akustischem Wege kann eine ausreichend tiefe Temperatur beispielsweise durch einen Piepton angezeigt werden, der endet, wenn der Kühlstab zu warm wird.

Durch das erfindungsgemäße Gerät wird vermieden, daß der behandelnde Arzt seine Aufmerksamkeit teilen muß zwischen dem zu behandelnden Objekt und einer Temperaturmeßeinrichtung.

Da sich erfindungsgemäß Temperaturmeßgerät und Temperaturanzeige im beziehungsweise am Griff befinden, entfällt jegliche Kabelverbindung und damit das umständliche Entfernen der Kabelverbindung und Einlegen des Kühlstabes in ein Isolierrohr. Wenn der Kühlstab und der Griff vorteilhafterweise so gestaltet werden, daß der Kühlstab durch leichten Druck bzw. Zug in den Griff einklinkbar bzw. abziehbar ist, kann der behandelnde Arzt diese Handlungen mit einer Hand vornehmen. Das Abziehen des Kühlstabes kann z.B. so erfolgen, daß er durch einen Abzugsring am Rande des Gefäßes mit Flüssigstickstoff festgehalten wird, von dem er nach Herauslösen des Kühlstabes aus dem Griff in das Gefäß mit flüssigem Stickstoff fällt. Das Einklinken des Kühlstabes in den Griff kann auf ähnlich einfache Weise bewerkstelligt werden, in-dem der Griff auf das aus dem Gefäß mit Flüssigstickstoff herausragende freie Ende des Kühlstabes gedrückt wird. Beim Einklinken wird gleichzeitig eine elektrisch leitende Verbindung zwischen dem Temperaturfühler in der Spitze des Kühlstabes und dem Temperaturmeßgerät im Innern des Griffes hergestellt. Dieses Temperaturmeßgerät besteht aus einer Platine mit elektronischen Bauteilen. Das Gerät kann so ausgebildet werden, daß unmittelbar nach dem Einklinken des Kühlstabes die Temperaturanzeige aktiviert wird. Diese Aktivierung kann aber auch durch einen Mikrotaster am Griff ausgelöst werden.

Die leichte Auswechselbarkeit des Kühlstabes mit nur einer Hand ist ein weiterer Vorteil des erfindungsgemäßen Gerätes. Sie ist besonders dann von Vorteil, wenn sich die Behandlund der Warze über mehrere Minuten erstreckt und mehrfach frisch gekühlte Kühlstäbe auf die Warze gedrückt werden müssen. Der Austausch der Kühlstäbe kann dann so schnell erfolgen, daß keine nennenswerte Unterbrechung der Behandlung erfolgt.

Entscheidend für die Eindringtiefe der Kälte in die Warze ist neben dem Durchmesser des Kühlstabes die Dauer, mit welcher der Kühlstab auf die Warze gedrückt wird. Bei einer vorteilhaften Ausgestaltung des erfindungsgemäßen Gerätes ist daher auf dem Griff zusätzlich eine Einrichtung zur Zeitmessung angeordnet. Diese Einrichtung kann in die Platine mit den elektronischen Bauteilen für die Temperaturmessung integriert werden. Für kürzere Behandlungsdauern genügt es hierfür, beispielsweise eine alle 5 Sek. blinkende Leuchtdiode vorzusehen. Für Behandlungsdauern von mehr als 1 Min. ist es dagegen günstiger, zur Zeitmessung eine Quarzuhr in den Griff einzubauen, welche die abgelaufene Behandlungszeit anzeigt. Falls gewünscht, kann auch eine Hilfseinrichtung vorgesehen werden, mit der eine gewünschte Behandlungsdauer vorprogrammiert wird. Das Ende der vorprogrammierten Behandlungsdauer wird durch ein optisches oder akustisches Signal angezeigt.

Mit Hilfe des erfindungsgemäßen Gerätes wird die Entfernung von Warzen so einfach, daß diese auch durch angelerntes Personal erfolgen kann. Für medizinisch unterversorgte Länder ist dies ein beachtlicher Vorteil, dies um so mehr, als gerade in solchen Ländern Warzen ein weitverbreitetes Leiden sind.

Die Zeichnungen veranschaulichen ein Ausführungsbeispiel der Erfindung.

Es zeigen:

Fig.1    einen Schnitt durch einen pistolenähnlichen Griff gemäß der Erfindung,

Fig.2    einen Schnitt durch einen Kühlstab
         mit einer Steckverbindung.

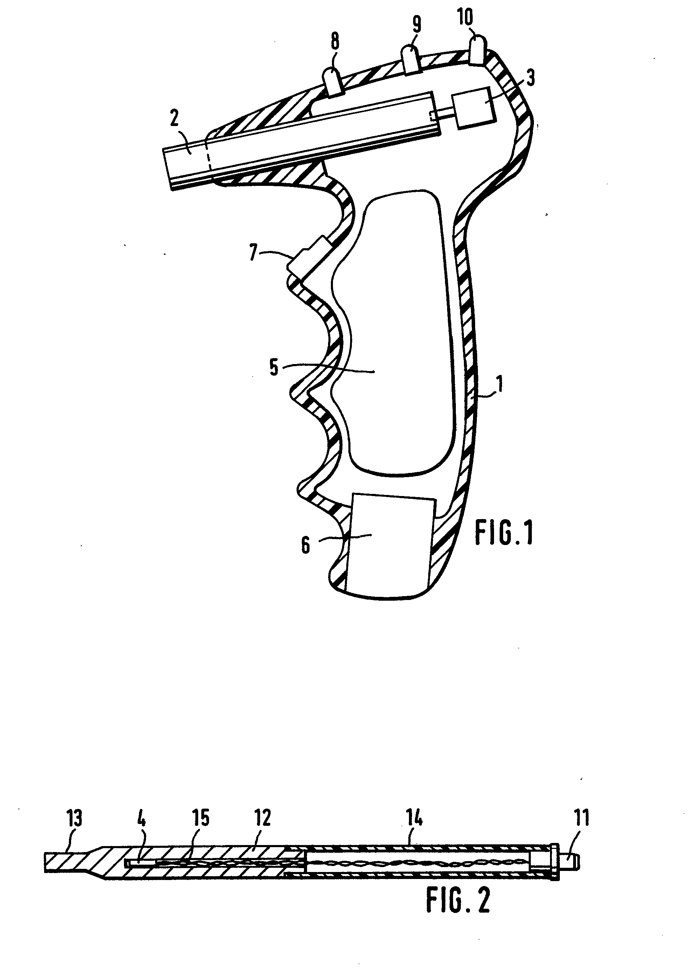

Der in Fig.1 dargestellte pistolenähnliche Griff 1 dient als Haltevorrichtung für den Kühlstab. Er besteht vorzugsweise aus Kunststoff oder eine Gußmasse auf der Basis von Kunststoff, z.B. aus Kunststoffpulver, Aluminiumpulver, Glasfaserwolle, flüssigem Kunstharz und einem Härter. Im oberen Teil des innen hohlen Griffes 1 befindet sich die Rohrführungshülse 2 zur Aufnahme des Kühlstabes. Die Rohrführungshülse 2 endet in einer Steckverbindung 3, in welche der Kühlstab beim Einsetzen einklinkt. Mit dem Einklinken wird der Temperaturfühler 4 des in Fig.2 dargestellten Kühlstabes an die elektronische Temperaturmeßeinrichtung im Innern des Griffes 1 angeschlossen. Diese Temperaturmeßeinrichtung ist nur schematisch angedeutet und besteht im wesentlichen aus einer Platine 5 mit elektronischen Bauteilen. Die Stromversorgung erfolgt durch eine im Batteriefach 6 befindliche Batterie. Durch Niederdrücken des Mikrotasters 7 wird der Stromkreis geschlossen und das Gerät aktiviert. Die Temperaturanzeige erfolgt durch eine rote Leuchtdiode 8 und eine grüne Leuchtdiode 9. Solange der in das Gerät gesteckte Kühlstab zu warm ist, gibt die Leuchtdiode 8 ein rotes Blinklicht ab. Ist dagegen die Temperatur des Kühlstabes ausreichend niedrig, blinkt die grüne Leichtdiode 9 auf. Die hierfür erforderliche Schaltung der elektronischen Bauteile auf der Platine 5 ist bekannt und daher nicht im einzelnen dargestellt.

Zur Zeitkontrolle ist noch eine gelbe Leuchtdiode 10 vorgesehen, die alle fünf Sek. aufblinkt, so daß sich der behandelnde Arzt durch Zählen der Blinkvorgänge über die abgelaufene Behandlungszeit informieren kann. Die für die Leuchtdiode 10 erforderlichen Bauteile sind in die Platine 5 integriert.

- 7 -

Der in Fig.2 dargestellte Kühlstab besitzt eine Steckverbindung 11 als Gegenstück zur Steckverbindung 3 des Griffes 1. Der eigentliche Kühlstab besteht aus einem teilweise hohlen Kupferstab 12, dessen Spitze 13 verjüngt ist. Im Hohlraum des Kupferstabes 12 befindet sich der Temperaturfühler 4, beispielsweise ein Widerstandsthermometer. Der Kupferstab 12 ist durch ein Kunststoffrohr 14 mit der Steckverbindung 11 verbunden. Der Temperaturfühler 4 ist durch ein Kabel 15 an die Steckverbindung 11 angeschlossen.

Zum Arbeiten mit dem erfindungsgemäßen Gerät werden mehrere Kühlstäbe bereit gehalten. Diese Kühlstäbe können auch Spitzen 13 mit unterschiedlichen Durchmessern haben. Die Kühlstäbe werden so in ein Gefäß mit flüssigem Stickstoff gestellt, daß die Kupferstäbe 12 völlig vom Stickstoff umgeben sind. Die Kühlstäbe sind kalt genug, sobald die heftige Stickstoffverdampfung aufgehört hat. Nunmehr wird der Griff 1 mit dem gewünschtem Kühlstab verbunden, in dem man die Rohrführungshülse 2 über den Kühlstab schiebt, bis die Steckverbindungen 3 und 11 durch Einklinken miteinander verbunden sind. Der Kühlstab sitzt nun fest im Griff 1 und kann aus dem Gefäß mit Flüssigstickstoff herausgenommen werden. Durch Niederdrücken des Mikrotasters 7 wird der Stromkreis zwischen Temperaturfühler 4, Platine 5, der Batterie im Batteriefach 6 und den Leuchtdioden 8, 9 und 10 geschlossen. Die entsprechenden Kabelverbindungen sind in Fig.1 nicht dargestellt worden. Die Leuchtdiode 9 gibt ein grünes Blinklicht ab, welches anzeigt, daß der Kupferstab 12 eine ausreichend tiefe Temperatur besitzt, beispielsweise zwischen -196 °C, der Temperatur des flüssigen Stickstoffs, und -150 °C. Solange der Mikrotaster 7 heruntergedrückt ist, leuchtet gleichzeitig die Leuchtdiode 10 alle fünf Sek. gelb auf. Die Entfernung der Warze kann nun beginnen, in dem der Kühlstab mit der Spitze 13 auf die Warze aufgesetzt wird.

Die Behandlungsdauer wird mit der Leuchtdiode 10 kontrolliert. Sobald der Kühlstab wärmer geworden ist als -150 °C erlischt die grüne Leuchtdiode 9 und die Leuchtdiode 8 blinkt rot. Der Kühlstab muß nun abgezogen werden, was auf einfache Weise mit Hilfe eines Abzugsringes am Behälter mit Flüssigstickstoff erfolgen kann. Der Kühlstab fällt in den Behälter zurück und ein ausreichend kalter neuer Kühlstab wird, wie beschrieben, in die Rohrführungshülse 2 eingeführt. Zum Auswechseln der Kühlstäbe werden nur wenige Sek. benötigt, so daß die Behandlung der Warze nicht nennenswert unterbrochen wird.

Ffm., 01.10.1980
Ba/Hi

0051747

Patentansprüche

1. Gerät zur Entfernung von Warzen mit Flüssigstickstoff, bestehend aus einem metallischen Kühlstab, in dessen Innerem ein Temperaturfühler angeordnet ist, der mit einem elektrischen Temperaturmeßgerät verbindbar ist, sowie einer Haltevorrichtung, in die der Kühlstab auswechselbar einsetzbar ist, gekennzeichnet durch folgende Merkmale:

   a) die Haltevorrichtung ist als pistolenähnlicher Griff (1) ausgebildet,

   b) das Temperaturmeßgerät (5) ist im Innern des Griffes angeordnet,

   c) die Temperaturanzeige (8,9) ist auf dem Griff angeordnet,

   d) der Temperaturfühler (4) des eingesetzten Kühlstabes ist mit dem Temperaturmeßgerät elektrisch leitend verbunden.

2. Gerät nach Anspruch 1, dadurch gekennzeichnet, daß die Temperaturanzeige durch zwei Leuchtdioden (8,9) mit den Anzeigebereichen "Temperatur ausreichend" und "Temperatur nicht ausreichend" erfolgt.

3. Gerät nach Anspruch 1, dadurch gekennzeichnet, daß die Temperaturanzeige durch ein akustisches Signal für den Anzeigebereich "Temperatur nicht ausreichend" erfolgt.

4. Gerät nach einem der Ansprüche 1 bis 3, dadurch gekennzeichnet, daß auf dem Griff eine Einrichtung (10) zur Zeitmessung
   angeordnet ist.

5. Gerät nach Anspruch 4, dadurch gekennzeichnet, daß die Einrichtung zur Zeitmessung eine in einem bestimmten Zeittakt blinkende Leuchtdiode (10) ist.

6. Gerät nach Anspruch 4, dadurch gekennzeichnet, daß die Einrichtung zur Zeitmessung eine in einem
   bestimmten Zeittakt ertönende Tonquelle ist.

7. Gerät nach Anspruch 4, dadurch gekennzeichnet, daß die Einrichtung zur Zeitmessung eine Uhr ist.

8. Gerät nach einem der Ansprüche 1 bis 7, dadurch gekennzeichnet, daß der Kühlstab durch leichten Druck bzw. Zug in den
   Griff einklinkbar bzw. abziehbar ist.

Ffm., 01.10.80

Ba/Hi

EM 980

MG 1252

0051747

FIG.1

FIG. 2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE – A – 2 037 900 (LENZ)<br>* Ansprüche 1,2; Figur 2 * | 1 |
| A | DE – A – 2 258 333 (GENZE)<br>* Seite 3, Zeile 26 – Seite 4, Zeile 1; Seite 4, Zeile 31 – Seite 5, Zeile 2; Figuren 1,2 * | 1 |
| A | DE – A – 2 214 703 (DRENSKE)<br>* Seite 3, Zeilen 4-34; Figuren * | 1-3 |
| A | GB – A – 2 026 324 (BRACCO)<br>* Seite 1, Zeilen 73-98; Seite 3, Zeilen 84-98 *<br>& DE – A – 2 925 270 | 4,7 |
| A | US – A – 3 823 575 (PAREL)<br>* Ansprüche 1,10,11 * | 1,2 |
| A | US – A – 4 174 631 (HAMMERSLAG)<br>* Spalte 2, Zeilen 29-39; Figur 1 * | 1 |
| A | US – A – 4 121 462 (MOHRMAN)<br>* Ansprüche 1-6 * | 2-4,6 |

**KLASSIFIKATION DER ANMELDUNG (Int. Cl.³)**

A 61 B 17/36

**RECHERCHIERTE SACHGEBIETE (Int. Cl.³)**

A 61 B
G 01 K

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| | | | |
|---|---|---|---|
| X | Der vorliegende Recherchenbericht wurde für alle Patentansprüche erstellt. | | |
| **Recherchenort**<br>Den Haag | **Abschlußdatum der Recherche**<br>09-02-1982 | **Prüfer**<br>BARTLETT | |